# EUROPEAN PATENT SPECIFICATION

(11) **EP 2 361 227 B1**
(45) Date of publication and mention of the grant of the patent: **05.03.2014**
(21) Application number: 09776202.5
(22) Date of filing: 29.09.2009
(51) Int. Cl.: C02F 1/467, C25B 9/08, C25B 15/02, A61L 2/03, A61L 2/18, C02F 5/02, C02F 5/08, C02F 1/461

(54) **DISINFECTION SYSTEM**
DESINFEKTIONSYSTEM
SYSTÈME DE DÉSINFECTION

(30) Priority: 30.09.2008 DK 200801364
(43) Date of publication of application: 31.08.2011
(73) Proprietor: Danish Clean Water A/S, 6430 Nordborg (DK)
(72) Inventor: JEPSEN, Hardy, DK-6400 Sønderborg (DK)
(74) Representative: Inspicos A/S
(86) International application number: PCT/DK2009/000213
(87) International publication number: WO 2010/037389

(56) References cited:
- EP-A- 0 612 694
- EP-A- 1 382 573
- WO-A-2008/089120
- JP-A- 6 238 275
- JP-A- 9 019 689
- JP-A- 2000 033 374
- JP-A- 2002 035 751
- JP-A- 2003 094 059
- US-A- 6 149 780
- US-A1- 2006 054 510
- US-B1- 6 200 434

## Description

This invention relates to a device for disinfection or sterilization of any kind of water where bacterial count has to be controlled such as industrial food processing systems like pasteurizers, where a disinfecting fluid containing an aqueous solution of sodium chloride of an electrochemically processed or produced anolyte and an electrochemically processed or produced catholyte, is feed to the food processing system, and where the disinfecting fluid is produced by electrolysis in a diaphragm electrochemical cell from an aqueous solution containing sodium chloride.

### Background of the invention

When an aqueous solution containing sodium chloride is electrolysed in an electrochemical cell, the type and the amount of electrolysis products are determined by several different parameters. These parameters include, among others, the initial concentration of sodium chloride in the aqueous solution to be electrolysed, the presence of impurities in said aqueous solution and the actual size of the applied potential. Another important parameter is whether a diaphragm separates the anode and the cathode.

The electrochemical processed of water involves the exposure of water and natural (or added) salts to a substantial difference in electrical potential. If an anode (+) and cathode (-) are placed in pure water and a direct current is applied, electrolysis of water occurs at the electrodes, leading to the breakdown of water into its constituent elements - gaseous oxygen and hydrogen.

Electroplating is a similar process, where chromium salts are added to water, a difference in potential is applied, and the chromium is deposited onto the material attached to the cathode. If sodium chloride (NaCl), or table salt, is used as a solution, the dominant electrolysis end product is various forms of chlorine and sodium hydroxide.

In US 3,616,355 an electrolysis process for generating aqueous solutions having enhanced biocidal activity is disclosed. In the method an aqueous solution of sodium chloride is electrolysed in an electrochemical cell, in which no diaphragm separates the anode and the cathode. It is described how the nature of the electrolysis products changes significantly when the voltage is increased to above about 10 volts in comparison to the products released when operating in the range of 3.5 to 7 volts, where chlorine and hypochlorine are the major products. Even though the full nature of this change is not understood, it appears to involve the generation of free radicals and/or charged or ionic species of varying stability, which appreciably modify and extend the biocidal activity of the cell effluent.

In the recent decades electrochemically processed or produced solutions have been produced in a diaphragm cell. Hence, an anolyte is produced at the anode in the anode chamber, which is separated from the cathode chamber by the diaphragm. The catholyte is produced at the cathode. Subsequently, the catholyte and the anolyte may be used separately or as a mixture.

It is well-known that the anolyte may be used as a biocide to kill bacteria, algae and fungi in drinking water and industrial water. This effect is assigned to the presence of H2O2, ClO2, HClO, HClO3, HCl, Cl2, O2, O3, H+, H3O+, CIO-, CI-, and free radicals. The catholyte may be used as a cleaning and/or bleaching solution. Typically the catholyte contains species, as HO2- and OH-. Due to the electrochemical reactions, which occur at each of the two electrodes, the final pH of the anolyte will be acidic whereas the final pH of the catholyte will be alkaline. Hence, in some cases it may be advantageous to mix the anolyte and the catholyte in order to produce a solution of neutral pH. Such mixed solutions may be used as disinfectants or sterilizing solutions.

In WO 00/24265 and WO 00/24275 an anolyte and a catholyte are produced in a diaphragm electrochemical cell by electrolysing an about 3 to 10% aqueous NaCl solution. These solutions are used for bacterial treatment of sausage castings and for bactericidal treatment of bio-film in food storage containers.

In US 2005/0194261 a solution useful as a corrosion inhibitor, cleaning agent, anti-scaling agent and as an inhibitor of sulphur reducing bacteria is produced. This solution is prepared by electrolysis of an aqueous solution containing about 10% NaCl. In the script it is clarified that the corrosion inhibiting effect is assigned to the fact that electrolysed solutions have the ability to kill the sulphur reducing bacteria clinging to the surface of metal items.

One major disadvantage of the above mentioned processes is that the electrodes, in particular the anode, very easily deteriorate as a result of corrosion due to the presence of chloride ions, when the electrode potential reaches a certain level. However, the recent development in improved electrode stability has made it possible to perform the electrolysis at markedly higher potentials.

A number of documents describe such electrochemical cells in apparatuses with different regulation mechanisms, such as for example US2006076248 disclosing an apparatus for producing electrolyzed water that includes an electrochemical cell and a solution reservoir containing a solution and having an outlet. An injection pump has an input end in fluid communication with the outlet of the solution reservoir and an output end in fluid communication with the electrochemical cell, wherein the injection pumps an amount of the solution from the solution reservoir to mix with a water solution and enter the electrochemical cell. A current feedback sensor senses a cell current in the electrochemical cell. A current control unit in data communication with the current feedback sensor and the injection pump wherein the current control unit compares the cell current with a preselected current and adjusts the amount of solution pumped from the injection pump responsive to the comparison. The apparatus may also include an automatic control feedback system to monitor and adjust pH, where the pH of the acidic EO output water is measured by a pH sensor located in the anolyte discharge conduit, which provides the feedback of actual pH to a pH controller. The controller then automatically adjusts the speed of a blend pump to control the actual pH to the pH setpoint of the controller. The blend pump is connected to output line and discharges to a mixing chamber wherein the recirculated catholyte is thoroughly mixed with the water from the water source and the solution from reservoir. Sensor, pump, and mixing chamber comprise the automated pH control loop.

Another example of the state of the art is US 2006/0054510.

In the described embodiment of the invention, the object is to introduce a disinfection system and a method for disinfection or sterilization especially (but not limited to) of industrial food processing devices, such as pasteurizers.

### Summery of the invention

It is an object of this invention to introduce a sterilization or disinfection system, especially for, but not limited to, industrial food processing devices or system, where the system will operate with a minimum of caretaking by the user, the user basically just having to ensure new supplies of substances used by the system.

It is a further object of this invention to introduce a sterilization or disinfection system that penetrates even biofilms to give sufficient but still cheap sterilization.

It is yet another an object of this invention to introduce a sterilization or disinfection system that is easily installed and maintained and is not significantly affected by the water temperature, but where bacteria are killed and any recolonization is controlled.

All of these objects are obtained by the surprising observation that, when an aqueous solution of sodium chloride to an amount of 5 to 10 g/l, or even up to 15 g/l or more, is electrolysed in a diaphragm electrochemical cell at a potential ranging from 15 to 25 V, an electrochemically processed or produced anolyte and an electrochemically processed or produced catholyte (in the following referred to as the disinfecting fluid) is produced, which, when used in combination, shows excellent properties in the form of disinfecting efficacy. These excellent properties are primarily assigned to the generation of active chlorine, which may be present at concentrations up to 2.000 mg/l. Various studies have revealed that these solutions are effective in combating waterborne pathogens and other microorganisms present in water, biofilms, and scales in water distribution systems as well as pathogens present in air.

Another advantage is the relatively low initial concentration of sodium chloride. Because of said low concentration the toxicity of the electrochemically processed or produced anolyte and catholyte is markedly lower than the toxicity of known electrochemically processed or produced electrolytes. Consequently, the electrolytes are friendlier towards the environment when disposed of after use.

All water disinfection will result in the formation of by-products and the invention is no exception, but will oxidize organic material to form lower levels of chlorates, thus reducing halogenated by-products. The inorganic by-products, (trihalomethanes -THMs, chlorite and chlorate) formed when the invention is used, are held in balance at much lower levels. Thus, smaller amounts of disinfection by-products are produced in the process, about 30% to 50% compared with sodium hypochlorite and other oxidants.

The disinfecting fluid is formed by introducing a disinfection system as specified in claim 1.

To minimize the number of components needed in the system, the system unlike for example

US2006076248 comprises no pH sensor, instead, the system further comprises a table programmed into the system, the table giving a relation, or dependence, of the pH value of the disinfection fluid to at least two parameters (such as, but not limited to, the pH value itself and a flow rate, or alternatively, the opening status of a valve), where table is used to regulate pH value of the disinfection fluid.

To regulate the pH of the disinfection fluid, the disinfection system further comprises means for communicating a determined fraction of electrochemically processed catholyte formed in cathode chamber to the anode chamber.

In the present invention the pH value of the disinfection fluid is regulated by leading a first fraction of the electrochemically processed catholyte formed in cathode chamber to the anode chamber by a second fluid communication having a first branch making fluid communication between the cathode chamber and the anode chamber, and a second fraction may be lead away from the diaphragm electrochemical cell by a second branch of the second fluid communication, this second fluid communication forming the means for communicating a determided fraction of electrochemically processed catholyte formed in cathode chamber to the anode chamber.

In the present invention, the sole fluid supply to the anode chamber is through the first branch of the second fluid communication. This is unlike systems like for example disclosed in US2006076248 where the water solution supply conduit is branched into two supplies, one for the cathode chamber and one for the anode chamber.

The parameters for the table in the preferred embodiment may for example include any number and permutation of:
- the concentration and flow rate of the mixed water,
- the voltage applied to the electrochemical cell (2), and
- the electrochemically processed catholyte formed in the cathode chamber (3) being feed to the anode chamber (4) (a value possible to deduce knowing the flows, voltages, concentrations etc. in the system).

Further, the disinfection system comprises a mixing manifold to mix different substances with water from a single water supply.

First of all, the first fluid communication is fluidically connected to a mixing manifold, the mixing manifold further being at least fluidically connected to a water supply and a salt reservoir through a third fluid communication, a controllable first pump being connected to the third fluid communication to pump saltwater, being formed by a mix of salt and water from the water supply and a salt reservoir, and where the controllable first pump is able to pump the saltwater at at least two different flow rates.

Secondly in one embodiment of the present invention, the mixing manifold further is fluidically connected to an acid supply through a fifth fluid communication, and where a controllable second pump pumping acid through the fifth fluid communication to the mixing manifold, where it is mixed with the softened water.

In order to ensure a correct concentration of the fluids entering the cathode chamber, in one preferred embodiment of the present invention, a conductivity sensor is positioned at the first fluid communication or in the mixing manifold measuring the conductivity of the mixed fluid flowing in the first fluid communication, this being a direct function of the salt concentration of the mixed fluid.

In one embodiment this conductivity measurement may be used to control the controllable first pump, increasing the flow rate when the conductivity, or salt concentration, gets below a set point salt concentration, this being an indication of the salt reservoir being close to empty, then optionally the system would give a signal or an alarm to refill the salt reservoir.

In another preferred embodiment the disinfection system further comprises a water softening device connected to a water supply feeding water to the softening device, the water softening devices softening the water by at least removing a substantial fraction of the chalk or calcium in the water, and feeding this softened through the fourth fluid communication to the mixing manifold where it is mixed with the saltwater.

A further objective of the present invention is to introduce an improved control of the pH value of the disinfecting fluid without introducing a sensor that has to be calibrated regularly. Therefore, since this pH value can be calculated with a substantially good certainty, given the parameters, such as the concentration and/or flow rate of the mixed water, the voltage applied to the electrochemical cell and and/or the electrochemically processed or produced catholyte formed in the cathode chamber being feed to the anode chamber, a table may be established and programmed into the system for these relations. The second fluid communication then further has a second branch and a controllable valve able to control the flow rate in the second branch, where the branch is connected to the externals or an reservoir. Based on such a table, the disinfection system uses a method to regulate the pH value of the disinfecting fluid by regulating the fraction of electrochemically processed or produced catholyte being transferred to the anode chamber from the cathode chamber through the second fluid communication. The second branch of the second fluid communication comprises a first controllable valve, so that this first controllable valve may increase and decrease the flow resistance of the second branch, thereby controlling the fraction of the electrochemically processed or produced catholyte that will flow through the second branch, and the fraction that will flow through the first branch and into the anode chamber. In this manner, a first fraction of the electrochemically processed or produced catholyte formed in the cathode chamber may be lead to the anode chamber and a second fraction may be lead away from the electrochemical cell, and the pH value of the disinfecting fluid is regulated by increasing the second fraction and correspondingly decreasing the first fraction, if the pH value is above the set point pH value, and decreasing the second fraction and correspondingly increasing the first fraction if the pH value is below the set point pH value.

One advantage by estimating the pH value based on a table and a number of parameters, is that a pH sensor is avoided in the system, where such a sensor would have to be calibrated and even exchanged from time to time. Further advantages that it improves the process of changing the desired pH level. Using a pH sensor would imply a feedback procedure, with a repeaded changing of the operational parameters and measuring of the resulting pH value, untill the desired value is obtained.With the present invention, the system by the table will know how to change the parameters to obtain the desired pH value. The main calibration needed for the system, will be when installed to calibrate it to the externals whereto it is connected, such as the dimensions of the tubes feeding liquids to the system.

### Figures:

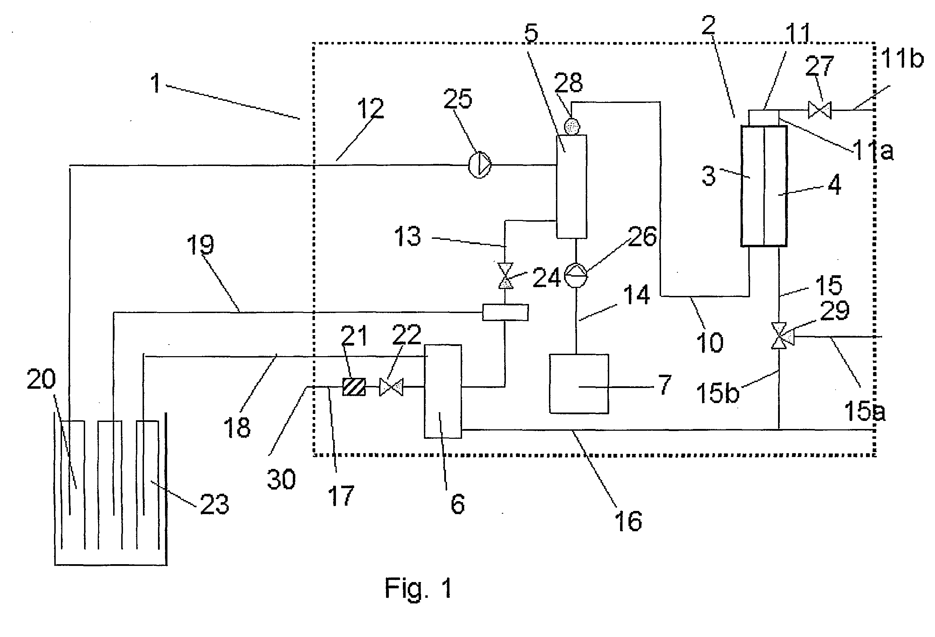

Fig. 1 shows a schematic view of the disinfection system of the invention.

### Detailed description

Fig. 1 is an illustration of the disinfection system (1) of the invention. The system comprises and electrochemical cell (2) being a diaphragm electrochemical cell, having a cathode chamber (3) and an anode chamber (4). The system further comprises a mixing manifold (5), a softening device (6), and an acid supply such as an acid reservoir (7).

A first fluid communication (10) forms a fluidic connection between the cathode chamber (3) and the mixing manifold (5). A second fluid communication (11) is fluidically connected to the cathode chamber (3), and has a first branch (11a) fluidically connected to the anode chamber (4), and a second branch (11 b) fluidically connected to the externals or a reservoir.

The mixing manifold (5) is fluidically connected to a salt and water supply (20) through a third fluid communication (12), to the softening device (6) through a fourth fluid communication (13), and to the acid supply (7) through a fifth fluid communication (14).

A sixth fluid communication (15) is fluidically connected to the anode chamber (4) and has a first branch (15a) fluidally connected to the externals, to an reservoir or to the system to be disinfected, and has a second branch (15b) merging with a seventh fluid communication (16), where the seventh fluid communication (16) forms fluidic connection from the softening device (6) and the externals, or a drain.

The softening device (6) is fluidically connected to a water supply through an eighth fluid communication (17).

The system operates by water entering from a main water supply (30) to the softening device (6) through the eighth fluid communication (17) the filter (21) and a first pressure regulating device (22) ensuring that the water is feed to the softening device (6) at a maximum predetermined pressure, the maximum predetermined pressure preferably being in the range of 2.5 to 5 bar, or more preferable in the range of 3 to 4 bar, or more preferably 3.3 bar. The softening device is connected to a softener supply (23) through a ninth communication (18), such as a fluid communication. Softener and water are mixed in the softening device (6) removing hardening elements such as chalk or calcium from the water. This softened water then leaves the softening device through the fourth fluid communication (13) and the remains from the softening process are removed through the seventh fluid communication (16). Before entering the mixing manifold (5) the softened water runs through a first flow regulation device (24) ensuring that the softened water enters the mixing manifold (5) with a flow volume possible set by the actual size of cell (2). Alternatively in another embodiment, the device (24) is or additionally comprises a second pressure regulating device (24) ensuring that the softened water enters the mixing manifold (5) at a predetermined minimum pressure, the predetermined minimum pressure being in the range of 1 to 2.5 bar, or more preferably in the range of 2 to 2.3 bar, or more preferably 2.2 bar.

In the mixing manifold (5) the softened water is mixed with saltwater transported from the salt and water supply (20) by the controllable first pumping device (25) through the third fluid communication (12). This mixed fluid is then transported to the cathode chamber (3) through the first fluid communication (10). A conductivity sensor (28) is either positioned at the first fluid communication (10), at the inlet of the cathode chamber (3), or inside or at the outlet of the mixing manifold (5), and measures the conductivity of the mixed fluid flowing in the first fluid communication (10), this being a direct function of the salt concentration of the mixed fluid.

The measured conductivity, and thereby also the salt concentration, of the mixed fluid, is used to control the controllable first pump (25), increasing the pumping rate and thereby the flow rate of the saltwater flowing in the third fluid communication (12), if the conductivity, or salt concentration, gets below a salt concentration set point, and if needed, decreasing the pumping rate if the conductivity, or salt concentration, gets above the salt concentration set point. This pumping rate is then able to estimate when the salt reservoir is close to empty, and, if so, to give a signal or alarm to the user of the disinfection system (1) to add new salt to the water and salt supply (20).

In one preferred embodiment of the invention the system decalcifies at predetermined intervals. This is done by shutting down the supply of saltwater to the mixing manifold (5) and at the same time pumping acid from the acid supply (7) to the mixing manifold (5) by the second controllable pumping device (26) through the fifth fluid communication (14) for either a predetermined, estimated or measured interval of time. This has the advantage that a rather concentrated acid supply (7) may be used, the needed concentration being mixed inside the mixing manifold (5) with the softened water. This gives a number of advantages, such as lowering the needed frequency for a possible acid reservoir (7) to be refilled, but also that a variable acid concentration may be supplied at the decalcification process, depending on the need, the user desire and programming into the disinfection system (1) (possible by a tuning button and the like), calculation etc.

The voltage applied to the electrochemical cell (2) is maintained at a predetermined value, however, as the electrochemical cell (2) calcifies, the resistance increases, meaning that the supplied current decreases. This is measured, and the measurement is used to estimate the calcification of the diaphragm electrochemical cell (2) and starting a decalcification process as described above when some specified level of calcification is reached. The time interval of decalcification may depend on the time to get the measured current below some predetermined value, and the acid concentration may depend on parameters such as the gradient whereby the current drops during the running process, the gradient whereby the current increases during the decalcification process, the desires and programming of the user etc.

In one preferred embodiment of the present invention, the system comprises a buffer reservoir (not shown) fluidically connected to the sixth fluid communication (15), and may optionally comprise means for opening and closing this fluidic connection. During decalcification the stored disinfection fluid in the buffer reservoir is then feed to the system to be disinfected or sterilized, either directly by a fluid communication from the buffer reservoir, or be feeding the fluid from the buffer reservoir to the first branch (15a) of the sixth fluid communication (15). Any numbers of valves, fluid communications and the like needed to store, direct and redirect the fluids of such a buffer reservoir construction.

The pH value of the disinfecting fluid preferably needs to be within some span, such as between 5 and 10, or more preferred between 6 and 9, or more preferred between 6 and 8. The disinfection system (1) may have means for the user to define the desired pH value, or the set point pH value, of the disinfecting fluid. Alternatively this set point pH value is predefined in the system. Since this pH value can be calculated with a substantially good certainty, given the parameters, such as the concentration and flow rate of the mixed water, the voltage applied to the electrochemical cell (2) and the electrochemically processed catholyte formed in the cathode chamber (3) being feed to the anode chamber (4), a table may be established and programmed into the system for these relations.

Based on such a table, the disinfection system (1) uses a method to regulate the pH value of the disinfecting fluid by regulating the fraction of electrochemically processed or produced catholyte being transferred to the anode chamber (4) from the cathode chamber (3) through the second fluid communication (11). The second branch (11b) of the second fluid communication (11) comprises a first controllable valve (27) so that, this first controllable valve (27) may increase and decrease the flow resistance of the second branch (11 b), thereby controlling the fraction of the electrochemically processed or produced catholyte that will flow through the second branch (11 b), and the fraction that will flow through the first branch (11 a) and into the anode chamber (4). In this manner, a first fraction of the electrochemically processed catholyte formed in the cathode chamber (3) may be lead to the anode chamber (4) and a second fraction may be lead away from the electrochemical cell (2), and the pH value of the disinfecting fluid is regulated by increasing the second fraction and correspondingly decreasing the first fraction or decreasing the second fraction and correspondingly increasing the first fraction in relation to the present pH value relative to the set point pH value.,

The system could advantageously have a tenth fluid communication (19) creating fluidic connection from the main water supply (30) to the salt and water supply (20) and/or the softening supply (23), thereby being the water supply for these.

## Claims

1. Disinfection system (1) comprising;
- a diaphragm electrochemical cell (2) having an anode chamber (4) and a cathode chamber (3),
- a mixing manifold (5),
- a first fluid communication (10) forming fluidic communication from the mixing manifold (5) to the cathode chamber (3),
- a second fluid communication (11) fluidically connected to the cathode chamber (3) and having a first branch (11a) fluidically connected to the anode chamber (4) and a second branch (11b) fluidically connected to the externals or a reservoir, said first branch (11a) forming the sole fluid supply to the anode chamber (3),
- a third fluid communication (12) forming fluidic communication between the mixing manifold (5) and a water supply (30) and a salt reservoir (20),
- a sixth fluid communication (15) fluidically connected to the anode chamber (4) and having a first branch (15a) fluidically connected to either the externals, to a reservoir or to the system to be disinfected,
- a controllable valve (27) arranged in the second branch (11b) of the second fluid communication (11), said controllable valve (27) being able to control the flow rate in the second branch (11b), where the second branch (11b) is connected to the externals or a reservoir, thereby allowing the pH value of the disinfection fluid to be regulated by leading a first fraction of the electrochemically processed catholyte to the anode chamber (3), via the first branch (11a), and leading a second fraction via the second branch (11b), wherein, the system (1) further comprises a table programmed into the system (1), the table giving a relation, or dependence, of the pH value of the disinfection fluid to at least two parameters, the system (1) thereby being capable of estimating the pH value of the disinfection fluid based on the table and the parameters, and the table being used to regulate the pH value of the disinfection fluid.

2. Disinfection system (1) as in claim 1, wherein the first fluid communication (10) is fluidically connected to the mixing manifold (5), the mixing manifold (5) further being fluidically connected to a water supply (30) and a salt reservoir (20) through the third fluid communication (12), and where a controllable first pump (25) being connected to the third fluid communication (12) to pump saltwater, being formed by a mix of salt and water from the water supply (30) and salt reservoir (20), and where the controllable first pump (25) being able to pump the saltwater by at least two different flow rates.

3. Disinfection system (1) as in claim 2, wherein a conductivity sensor (28) is positioned at the first fluid communication (10) or in the mixing manifold (5) measuring the conductivity of the mixed fluid flowing in the first fluid communication (10), this being a direct function of the salt concentration of the mixed fluid.

4. Disinfection system (1) as in any of the preceding claims, wherein the pump rate is used to estimate when the salt in the salt reservoir (20) is close to empty, then giving a signal or alarm.

5. Disinfection system (1) as in claims 2-4, wherein the mixing manifold (5) is fluidically connected to a water softening device (6) through a fourth fluid communication system (13), the water softening device (6) further being connected to a water supply (30) feeding water to the softening device (6), and where the water softening device (6) softening the water by at least removing a substantial fraction of the chalk or calcium in the water, and feeding this softened water through the fourth fluid communication (13) to the mixing manifold (5) where it is mixed with the saltwater, and where the mixed fluid flowing in the first fluid communication (10) thus is at least a mix of the saltwater and the softened water.

6. Disinfection system (1) as in claim 5, wherein the mixing manifold (5) is further fluidically connected to an acid supply (7) through a fifth fluid communication (14), and where a controllable second pump (26) pumping acid through the fifth fluid communication (14) to the mixing manifold (5), where it is mixed with the softened water.

7. Disinfection system (1) as in claim 6, wherein the fourth fluid communication system (13) comprises a pressure switch comprising a pressure measuring device and is able to control the pressure of the fluid in the fourth fluid communication system (13).

8. Disinfection system (1) as in any of the preceding claims, wherein the current or voltage over the diaphragm electrochemical cell (2) is measured, and the measurement is used to estimate the calcification in the diaphragm electrochemical cell (2).

## Patentansprüche

1. Desinfizierungssystem (1) mit:
- einer elektrochemischen Membranzelle (2) mit einer Anodenkammer (4) und einer Kathodenkammer (3),
- einer Mischrohrverzweigung (5),
- einer ersten Flüssigkeitsverbindung (10) zur Flüssigkeitsförderung von der Mischrohrverzweigung (5) zur Kathodenkammer (3),
- einer zweiten Flüssigkeitsverbindung (11) in Flüssigkeitsverbindung mit der Kathodenkammer (3) und mit einer ersten Abzweigung (11a) in Flüssigkeitsverbindung mit der Anodenkammer und einer zweiten Abzweigung (11b) in Flüssigkeitsverbindung mit der Umgebung oder einem Behälter, wobei die erste Abzweigung (11a) die einzige Flüssigkeitsversorgung zur Anodenkammer (4) bildet,
- einer dritten Flüssigkeitsverbindung (12) zur Flüssigkeitsverbindung zwischen der Mischrohrverzweigung (5) und einer Wasserversorgung (30) und einem Salzbehälter (20),
- einer sechsten Flüssigkeitsverbindung (15) in Flüssigkeitsverbindung mit der Anodenkammer (4) und mit einer ersten Abzweigung (15a) in Flüssigkeitsverbindung mit entweder der Umgebung, einem Behälter oder dem zu desinfizierenden System,
- einem regelbaren Ventil (27) angeordnet in der zweiten Verzweigung (11b) der zweiten Flüssigkeitsverbindung (11) zur Regelung der Durchflussmenge in der zweiten Verzweigung (11b), wobei die zweite Verzweigung (11b) mit der Umgebung oder einem Behälter verbunden ist um eine Regelung vom pH-Wert der Desinfizierungsflüssigkeit durch Einspeisung eines ersten Bruchteils des elektrochemisch behandelten Katholyts in die Anodenkammer (4) durch die erste Verzweigung (11a) und Einspeisung eines zweiten Bruchteils durch die zweite Verzweigung (11b) zu erzielen, wobei das System (1) zusätzlich eine in das System (1) einprogrammierte Tabelle aufweist, die ein Verhältnis oder eine Abhängigkeit des pH-Wertes der Desinfizierungsflüssigkeit zu mindestens zwei Parametern gibt, wobei das System (1) imstande ist den pH-Wert der Desinfizierungsflüssigkeit auf Grund der Tabelle und der Parameter zu schätzen und die Tabelle zur Regelung des pH-Wertes der Desinfizierungsflüssigkeit angewandt wird.

2. Desinfizierungssystem (1) nach Anspruch 1, wobei die erste Flüssigkeitsverbindung (10) in Flüssigkeitsverbindung mit der Mischrohrverzweigung (5) ist, die zusätzlich über eine dritte Flüssigkeitsverbindung (12) mit einer Wasserversorgung (30) und einem Salzbehälter (20) in Flüssigkeitsverbindung steht, und eine regelbare erste Pumpe (25) mit der dritten Flüssigkeitsverbindung (12) zum Pumpen von Salzwasser verbunden ist, das aus einer Mischung von Salz und Wasser aus der Wasserversorgung (30) und dem Salzbehälter (20) gebildet wird, und wobei die regelbare erste Pumpe (25) imstande ist das Salzwasser mit mindestens zwei Durchflussmengen zu pumpen.

3. Desinfizierungssystem (1) nach Anspruch 2, wobei ein Leitfähigkeitssensor (28) an der ersten Flüssigkeitsverbindung (10) oder in der Mischrohrverzweigung (5) zur Messung der Leitfähigkeit der durch die erste Flüssigkeitsverbindung (10) fließende gemischte Flüssigkeit angeordnet ist, wobei die Leitfähigkeit eine direkte Funktion der Salzkonzentration der gemischten Flüssigkeit ist.

4. Desinfizierungssystem (1) nach jedem der vorhergehenden Ansprüche, wobei die Pumpenleistung angewandt wird, um zu schätzen ob der Salzbehälter (20) annähernd leer ist und dann ein Signal oder ein Alarm abzugeben.

5. Desinfizierungssystem (1) nach den Ansprüchen 2-4, wobei die Mischrohrverzweigung (5) durch ein viertes Flüssigkeitsverbindungssystem (13) in Flüssigkeitsverbindung mit einem Wasserenthärtungsgerät (6) ist, wobei das Wasserenthärtungsgerät (6) mit einer Wasserversorgung (30) zur Einspeisung von Wasser in das Enthärtungsgerät (6) verbunden ist, und das Wasserenthärtungsgerät (6) das Wasser durch Entfernen eines erheblichen Anteils vom Kalk oder Kalcium aus dem Wasser enthärtet, und das enthärtete Wasser durch die vierte Flüssigkeitsverbindung (13) zur Mischrohrverzweigung (5) führt, wo es mit dem Salzwasser vermischt wird und wobei die in der ersten Flüssigkeitsverbindung (10) fließende Flüssigkeit somit zumindest eine Mischung aus Salzwasser und enthärtetem Wasser ist.

6. Desinfizierungssystem (1) nach Anspruch 5, wobei die Mischrohrverzweigung (5) durch eine fünfte Flüssigkeitsverbindung (14) zusätzlich mit einer Säureversorgung verbunden ist, und wobei eine regelbare zweite Pumpe (26) Säure durch die fünfte Flüssigkeitsverbindung (14) zur Mischrohrverzweigung (5) pumpt, wo es mit dem enthärteten Wasser vermischt wird.

7. Desinfizierungssystem (1) nach Anspruch 6, wobei das vierte Flüssigkeitsverbindungssystem (13) einen Druckschalter mit einem Druckmessgerät aufweist und imstande ist den Druck der Flüssigkeit im vierten Flüssigkeitsverbindungssystem (13) zu regeln.

8. Desinfizierungssystem (1) nach jedem der vorhergehenden Ansprüche, wobei der Strom oder die Spannung über der elektrochemischen Membranzelle (2) gemessen wird und die Messung zur Schätzung der Kalkbildung in der elektrochemischen Membranzelle (2) angewandt wird.

## Revendications

1. Système de désinfection (1) comprenant:
- cellule électrochimique de diaphragme (2) ayant une chambre d'anode (4) et une chambre de cathode (3),
- un collecteur de mélange (5),
- une première communication fluide (10) formant une communication fluidique du collecteur de mélange (5) à la chambre de cathode (3),
- une seconde communication de fluide (11) raccordée de manière fluidique à la chambre de cathode (3) et ayant un premier circuit (11a) raccordé de manière fluidique à la chambre d'anode (4) et un second circuit (11b) raccordé de manière fluidique aux externes ou à un réservoir, ledit premier circuit (11a) formant l'unique alimentation en liquide vers la chambre d'anode (3),
- une troisième communication de fluide (12) formant une communication fluidique entre le collecteur de mélange (5) et une alimentation en eau (30) et un réservoir de sel (20),
- une sixième communication de fluide (15) raccordée de manière fluidique à la chambre d'anode (4) ayant un premier circuit (15a) raccordé de manière fluidique soit aux externes soit à un réservoir ou au système à désinfecter,
- une vanne contrôlable (27) disposée dans le second circuit (11b) de la seconde communication de fluide (11), ladite vanne contrôlable (27) étant apte à contrôler le débit dans le second circuit (11b), dans laquelle le second circuit (11b) est raccordé aux externes ou à un réservoir, permettant ainsi à la valeur de pH du liquide de désinfection d'être régulée en amenant une première fraction du catholyte traité électrochimiquement vers la chambre d'anode (3), par le premier circuit (11a), et en amenant une seconde fraction par le second circuit (11b),
dans laquelle, le système (1) comprend en outre un tableau programmé dans le système (1), le tableau donnant une relation, ou dépendance de la valeur de pH du liquide de désinfection vers au moins deux paramètres, le système (1) étant ainsi apte à estimer la valeur de pH du liquide de désinfection sur la base du tableau et des paramètres, et le tableau étant utilisé pour réguler la valeur de pH du liquide de désinfection.

2. Système de désinfection (1) tel que dans la revendication 1, dans lequel la première communication de fluide (10) est raccordée de manière fluidique au collecteur de mélange (5), le collecteur de mélange (5) étant en outre raccordé de manière fluidique à une alimentation en eau (30) et à un réservoir de sel (20) par la troisième communication de fluide (12), et dans lequel une première pompe contrôlable (25) étant raccordée à la troisième communication de fluide (12) pour pomper l'eau salée, étant formée par un mélange de sel et d'eau provenant de l'alimentation en eau (30) et du réservoir de sel (20), et dans lequel la première pompe contrôlable (25) étant apte à pomper l'eau salée par au moins deux débits différents.

3. Système de désinfection (1) tel que dans la revendication 2, dans lequel un capteur de conductivité (28) est positionné sur la première communication de fluide (10) ou dans le collecteur de mélange (5) mesurant la conductivité du liquide mélangé dans la première communication de fluide (10), ceci étant une fonction directe de la concentration en sel du liquide mélangé.

4. Système de désinfection (1) tel que dans l'une quelconque des revendications précédentes, dans lequel le débit de pompe est utilisé pour estimer quand le sel du réservoir de sel (20) est pratiquement vide, en donnant alors un signal ou une alarme.

5. Système de désinfection (1) tel que dans les revendications 2-4, dans lequel le collecteur de mélange (5) est raccordé de manière fluidique à un dispositif d'adoucissement de l'eau (6) par un quatrième système de communication de fluide (13), le dispositif d'adoucissement de l'eau (6) étant en outre raccordé à une alimentation en eau (30) alimentant en eau le dispositif d'adoucissement (6), et dans lequel le dispositif d'adoucissement de l'eau (6) adoucissant l'eau en enlevant au moins une fraction substantielle de craie ou de calcium dans l'eau, et alimentant cette eau adoucie par la quatrième communication de fluide (13) vers le collecteur de mélange (5) dans lequel elle est mixée à l'eau de mer, et dans lequel le liquide mixé s'écoulant dans la première communication de fluide (10) est donc un mélange de l'eau salée et de l'eau adoucie.

6. Système de désinfection (1) tel que dans la revendication 5, dans lequel le collecteur de mélange (5) est en outre raccordé de manière fluidique à une alimentation en acide (7) par une cinquième communication de fluide (14) et dans lequel une seconde pompe contrôlable (26) pompant l'acide par la cinquième communication de fluide (14) vers le collecteur de mélange (5) dans lequel il est mélangé avec l'eau adoucie.

7. Système de désinfection (1) tel que dans la revendication 6, dans lequel le système de communication de fluide (13) comprend un commutateur de pression comprenant un dispositif de mesure de la pression et est apte à contrôler la pression du liquide dans le quatrième système de communication de fluide (13).

8. Système de désinfection (1) tel que dans l'une quelconque des revendications précédentes, dans lequel le courant ou la tension sur la cellule électrochimique du diaphragme (2) est mesurée, et la mesure est utilisée pour estimer la calcification dans la cellule électrochimique du diaphragme (2).
